# EUROPÄISCHE PATENTSCHRIFT

(11) **EP 1 572 732 B1**
(45) Veröffentlichungstag und Bekanntmachung des Hinweises auf die Patenterteilung: **22.08.2007**
(21) Anmeldenummer: 03767664.0
(22) Anmeldetag: 26.11.2003
(51) Int. Cl.: C07K 14/435, A01K 67/00

(54) **ISOLIERTES FLUORESZIERENDES PROTEIN AUS CLYTIA GREGARIA CGFP, SOWIE DESSEN VERWENDUNG**
ISOLATED FLUORESCENT PROTEIN FROM CLYTIA GREGARIA CGFP AND USE THEREOF
PROTEINE FLUORESCENTE DE CLYTIA GREGARIA (CGFP) ISOLEE ET SON UTILISATION

(30) Priorität: 09.12.2002 DE 10257354
(43) Veröffentlichungstag der Anmeldung: 14.09.2005
(73) Patentinhaber: Bayer HealthCare AG, 51368 Leverkusen (DE)
(72) Erfinder: GOLZ, Stefan, 45326 Essen (DE); MARKOVA, Svetlana, Krasnoyarsk, 660036 (RU); BURAKOVA, Ludmila, Sosnovoborsk, 662500 (RU); FRANK, Ludmila, Krasnoyarsk, 660036 (RU); VYSOTSKI, Eugene, Krasnoyarsk, 660100 (RU)
(86) Internationale Anmeldenummer: PCT/EP2003/013281
(87) Internationale Veröffentlichungsnummer: WO 2004/052926

(56) Entgegenhaltungen:
- LEVINE L D ET AL: "ISOLATION AND CHARACTERIZATION OF A PHOTO PROTEIN PHIALIDIN AND A SPECTRALLY UNIQUE GREEN FLUORESCENT PROTEIN FROM THE BIO LUMINESCENT JELLYFISH PHIALIDIUM-GREGARIUM" COMPARATIVE BIOCHEMISTRY AND PHYSIOLOGY B, Bd. 72, Nr. 1, 1982, Seiten 77-86, XP009028577 ISSN: 0305-0491 in der Anmeldung erwähnt
- CHALFIE, M. AND KAIN, S.: "Green fluorescent protein: properties, applications, and protocols" August 1998 (1998-08) , WILEY-LISS, INC. XP009028583 in der Anmeldung erwähnt Seite 49; Abbildung 3.3 Seite 70
- PRASHER D C: "Using GFP to see the light" TRENDS IN GENETICS, ELSEVIER, AMSTERDAM, NL, Bd. 11, Nr. 8, 1995, Seiten 320-323, XP004207387 ISSN: 0168-9525
- TSIEN R Y: "THE GREEN FLUORESCENT PROTEIN" ANNUAL REVIEW OF BIOCHEMISTRY, PALTO ALTO, CA, US, Bd. 67, 1998, Seiten 509-544, XP000946725 ISSN: 0066-4154
- INOUYE SATOSHI ET AL: "Cloning and sequence analysis of cDNA for the calcium-activated photoprotein, clytin" FEBS (FEDERATION OF EUROPEAN BIOCHEMICAL SOCIETIES) LETTERS, Bd. 315, Nr. 3, 1993, Seiten 343-346, XP001180448 ISSN: 0014-5793
- PRASHER D C ET AL: "PRIMARY STRUCTURE OF THE AEQUOREA VICTORIA GREEN-FLUORESCENT PROTEIN" GENE, ELSEVIER, AMSTERDAM, NL, Bd. 111, 1992, Seiten 229-233, XP001018985 ISSN: 0378-1119 -& DATABASE GENBANK [Online] 26. April 1993 (1993-04-26) PRASHER ET AL.: "Primary Structure of the Aequorea victoria green-fluorescent protein" Database accession no. M62653 XP002276253
- CHALFIE M. ET AL: 'Green Fluorescent Protein as a Marker for Gene Expression' SCIENCE Bd. 263, 1994, Seiten 802 - 805, XP002003599
- INOUYE S.; TSUJI F.I.: 'Aequorea green fluorescent protein: Expression of the gene and fluorescence characteristics of the recombinant protein' FEBS LETTERS Bd. 341, 1994, Seiten 277 - 280, XP002030244

### SEQUENCE LISTING

<110> Bayer AG, BHC
<120> Isoliertes fluoreszierendes Protein CGFP, sowie dessen Verwendung
<130> Le A 36 493
<160> 2
<170> Patent In version 3.1
<210> 1
   <211> 708
   <212> DNA
   <213> Clytia gregaria
<400> 1
   atgactgcac ttaccgaagg agcaaaactg ttcgagaaag aaattcccta cattacagag     60
   ttggaaggag acgttgaagg aatgaaattc atcatcaaag gtgaaggtac tggcgacgct    120
   actactggca ccatcaaagc gaaatatatt tgcacaactg gtgaccttcc tgtaccatgg    180
   gctaccatct tgagtagttt gtcgtatggt gttttctgtt tcgctaagta tccacgccac    240
   attgccgact ttttcaagag cacacaacca gatggttatt cacaagacag aatcattagt    300
   tttgacaatg atggacaata cgatgtcaaa gccaaggtta cttatgaaaa cggaacactt    360
   tataatagag tcacagtcaa aggtactggc ttcaaatcaa acggcaacat ccttggtatg    420
   agagttctct accattcacc accacacgct gtctacatcc ttcctgaccg taaaaatggt    480
   ggcatgaaaa ttgaatacaa taaggctttc gacgttatgg gcggtggtca ccaaatggcg    540
   cgtcacgccc aattcaataa accactagga gcctgggaag aagattatcc gttgtatcat    600
   catcttaccg tatggacttc tttcggaaaa gatccggatg atgatgaaac tgaccatttg    660
   accatcgtcg aagtcatcaa agctgttgat ttggaaacat accgttga                 708
<210> 2
   <211> 235
   <212> PRT
   <213> Clytia gregaria
<400> 2

## Beschreibung

Die Erfindung betrifft die Nukleotid- und Aminosäuresequenz, sowie die Aktivität und Verwendung des fluoreszierenden Proteins CGFP (fluorescence protein of clytia gregaria).

### Fluoreszierende Proteine

Eine Vielzahl an Coelenteraten sind biolumineszent (Morin et al., - 1974) und emittieren blaues oder grünes Licht. Das 1962 als erstes Licht produzierendes Protein identifizierte Aequorin aus Aequoria victoria (Shimomura et al., 1962) emittierte als isoliertes Protein ein blaues Licht und nicht wie das phenotypisch beobachtete grüne Licht von Aequoria victoria. Später konnte das grün fluoreszierende Protein (GFP) aus Aequoria victoria isoliert werden, das Aufgrund der Anregung durch das Aequorin die Meduse phenotypisch grün erscheinen lässt (Johnson et al, 1962; Hastings et al., 1969; Inouye et al, 1994).

Grün fluoreszierende Proteine konnten aus unterschiedlichen Organismen isoliert werden. Hierzu zählen die Hydozoa (aequoria, halistaura obelia) und Anthropoden (acanthotilum, sea cactus, cavernularia, renila, ptilosarcus, stylatula) (Morin et al., 1971; Morin et al., 1971 II, Wampler et al., 1971, Wampler et al., 1973, Cormier et al., 1973, Cormier et al., 1974, Levine et al., 1982).

Eine Zusammenfassung einiger fluoreszierender Proteine findet sich in Tabelle 1:

**Tabelle 1:**

| Übersicht über einige fluoreszierende Proteine. Angegeben ist der Name, der Organismus aus dem das Protein isoliert worden ist und die Identifikationsnummer (Acc. No.) des Datenbankeintrages. | | |
|---|---|---|
| **Name** | **Organismus** | **Identifikations** Nr. |
| Green fluorescent protein | Aequorea macrodactyla | AF435433 |
| Green fluorescent protein | Aequoria | L29345 |
| Green fluorescent protein-like protein | Agaricia agaricites | AY037775 |
| Green fluorescent protein-like protein | Agaricia fragilis | AY037765 |
| Green fluorescent protein | Dendronephthya | AF420591 |
| Red fluorescent protein | Entacmaea quadricolor | AY130757 |
| Green fluorescent protein-like protein | Caribbean anemone | AY037777 |
| Green fluorescent protein | Heteractis crispa | AF420592 |
| Green fluorescent protein-like protein | Montastraea annularis | AY037766 |
| Green fluorescent protein-like protein | Montastraea cavernosa | AY037768 |
| Cyan fluorescent protein | Montastraea cavernosa | AY056460 |
| Green fluorescent protein | Renilla muelleri | AY015996 |
| Green fluorescent protein | Renilla renoformis | AF372525 |
| Green fluorescent protein-like protein | Ricordea florida | AY037774 |

Die fluoreszierenden Proteine unterscheiden sich nicht nur aufgrund ihrer Nukleotid-und Aminosäuresequenz, sondern auch aufgrund ihrer biochemischen und physikalischen Eigenschaften. Die spektralen Charakteristika der fluoreszierenden Proteine können sich sowohl auf der Exitations- als auch auf der Emmisionsseite unterscheiden. Eine Übersicht der Spektren der Fluoreszenz und der Anregungswellenlänge findet sich in Tabelle 2.

**Tabelle 2:**

| Übersicht über einige fluoreszierende Proteine. Angegeben ist der Organismus aus dem das Protein isoliert worden ist, die Anregungs- und Emissionswellenlängen, die bei Spektralanalysen bestimmt worden sind. | | |
|---|---|---|
| **Organismus** | **Anregung** | **Fluoreszenz** |
| Aequoria | 465-498 nm | 509 nm |
| Halistaura | 465 nm | 497 nm |
| Phialidium | 485 nm | 498 nm |
| Renilla | 498 nm | 508 nm |

Die Verwendung von fluoreszierenden Proteinen wurde bereits zuvor beschrieben. Eine Übersicht findet sich in Tabelle 3:

**Tabelle 3:**

| Übersicht über einige fluoreszierende Proteine. Angegeben ist der Organismus aus dem das Protein isoliert worden ist, der Name des fluoreszierenden Proteins und eine Auswahl an Patenten bzw. Anmeldungen. | | |
|---|---|---|
| **Organismus** | **Fluoreszierendes Protein** | **Patent / Anmeldung** |
| Renilla mulleri | GFP | US patent no. 6,436,682 |
| | | WO200168824 |
| | | WO200257451 |
| | | WO200134824 |
| | | WO9949019 |
| | | US patent no. 6,232,107 |

| **Organismus** | **Fluoreszierendes Protein** | **Patent / Anmeldung** |
|---|---|---|
| Aequoria | GFP | WO200071565 |
| | | WO9711094 |
| | | WO9623898 |
| | | US patent no. 5,958,713 |
| | | US patent no. 6,172,188 |

Es konnte gezeigt werden, dass durch die Veränderung der Aminosäuresequenz von fluoreszierenden Proteinen die physikalischen und biochemischen Eigenschaften verändert werden können. Beispiele von mutagenisierten fluoreszierenden Proteinen sind in der Literatur beschrieben (Delagrave et al., 1995; Ehrig et al., 1995; Heim et al., 1996).

Fluoreszierende Proteine finden bereits in unterschiedlichsten Gebieten eine Anwendung. Die Verwendung von fluoreszierende Proteinen beim 'Fluorescence Resonance Energy Tranfer'(FRET), 'Bioluminescence Resonance Energy Transfer (BRET) und anderen Energietransferverfahren wurde bereits in der Literatur beschrieben (Mitra et al., 1996; Ward et al., 1978; Cardullo et al, 1988; US patent no. 4,777,128; US patent no. 5,126,508; US patent no. 4,927,923; US patent no. 5,279,943). Weitere Nicht-radioaktive Methoden zum Energietransfer mittels GFP wurden in ebenfalls bereits beschrieben (PCT appl. WO 98/02571 and WO 97/28261)

### Reportersysteme

Als Reporter- oder Indikatorgen bezeichnet man generell Gene, deren Genprodukte sich mit Hilfe einfacher biochemischer oder histochemischer Methoden leicht nachweisen lassen. Man unterscheidet mindestens 2 Typen von Reportergenen.
1. Resistenzgene. Als Resistenzgene werden Gene bezeichnet, deren Expression einer Zelle die Resistenz gegen Antibiotika oder andere Substanzen verleiht, deren Anwesenheit im Wachstumsmedium zum Zelltod führt, wenn das Resistenzgen fehlt.
2. Reportergen. Die Produkte von Reportergenen werden in der Gentechnologie als fusionierte oder unfusionierte Indikatoren verwendet. Zu den gebräuchlichsten Reportergenen gehört die beta-Galaktosidase (Alam et al., 1990), alkalische Phosphatase (Yang et al., 1997; Cullen et al., 1992), Luciferasen und andere Photoproteine (Shinomura, 1985; Phillips GN, 1997; Snowdowne et al., 1984).

Als Lumineszenz bezeichnet man die Abstrahlung von Photonen im sichtbaren Spektralbereich, wobei diese durch angeregte Emittermoleküle erfolgt. Im Unterschied zur Fluoreszenz wird hierbei die Energie nicht von Außen in Form von Strahlung kürzerer Wellenlänge zugeführt.

Man unterscheidet Chemilumineszenz und Biolumineszenz. Als Chemolumineszenz bezeichnet man eine chemische Reaktion die zu einem angeregten Molekül führt, das selbst leuchtet, wenn die angeregten Elektronen in den Grundzustand zurückkehren. Wird diese Reaktion durch ein Enzym katalysiert, spricht man von Biolumineszenz. Die an der Reaktion beteiligten Enzyme werden generell als Luziferasen bezeichnet.

### Einordung der Spezies Clytia gregaria

Cnidaria->Leptomedusae→Campanulariidae→ Clytia gregaria

Die Spezies Clytia gregaria gehört zu den Cnidaria, speziell zu den Medusen. Der biolumineszente bzw. fluoreszente Phänotyp wurde bereits 1998 beschrieben (Ward et al., 1998).

### Isolierung der cDNA

Zur Untersuchung der fluoreszenten Aktivität der Spezies Clytia gregaria wurden Exemplare im Friday Harbor in Washington State (USA) gefangen und in flüssigem Stickstoff gelagert. Zur Herstellung der cDNA-Bibliothek wurde ausschließlich der biolumineszente Ring eines Medusenexemplars verwendet. Zur Erstellung der cDNA-Bibliotheken von Clytia gregaria, wurde die RNA nach der Methode von Krieg (Krieg et al., 1996) durch Isothiocyanat isoliert.

Zur Herstellung der cDNA wurde eine RT-PCR durchgeführt. Hierzu wurden 10 µg RNA mit Reverser Transkriptase (Superscribt Gold II) nach folgendem Schema inkubiert:

| | | | | |
|---|---|---|---|---|
| PCR | 1. | 30 | Sekunden | 95°C |
| | 2. | 6 | Minuten | 68°C |
| | 3. | 10 | Sekunden | 95°C |
| | 4. | 6 | Minuten | 68°C |

17 Zyklen von Schritt 4 nach Schritt 3

Die Reaktionsprodukte wurden zur Inaktivierung der Polymerase für 30 Minuten bei 37°C mit Proteinase K inkubiert und die cDNA mit Ethanol präzipitiert. Die cDNA wurde in Wasser gelöst und mit SfiI für eine Stunde bei 37°C inkubiert. Die Reaktionsprodukte wurden zur Abtrennung kleiner Fragmente gelfiltriert. Die fraktionierte cDNA wurde anschließend in den SfiI geschnittenen und dephosphorilierten λTriplEx2 Vector ligiert. Zur Herstellung einer λ-Phagen Expressionsbank wurden die klonierten cDNA-Fragmente anschließend durch das in vitro Verpackungssystem SMART cDNA Library Construction Kits (Clontech) in λ-Phagen verpackt.

Die Identifizierung der rekombinaten Phagen, die eine cDNA Insertion mit potentieller Expression von fluoreszenten Proteinen enthielten, wurde ein ,,library screening" durchgeführt.

Hierzu wurden Bakterienrasen aus transformierten E. coli XL1-Blue auf 90 mm Kulturschalen plattiert und für 12-15 Stunden bei 31°C inkubiert. Die Induktion der Proteinexpression durch die Zugabe von 60 µl einer 20 mM IPTG (Isopropythiogalactoside) Lösung auf die Platten gestartet. Nach einer Inkubation 24 Stunden bei Raumtemperatur wurden die Platten für 72 Stunden bei 4 °C gelagert. Anschliessend erfolgte die Messung der Fluoreszenz.

Hierzu wurden die Bakterien mit einem Argon-Laser (LGN502) mit 100 mV bei 488 nm oder 366 nm (UVL21) bestrahlt. Die Fluoreszenz wurde unter Verwendung eines 510 nm ZSV Filters gemessen.

Zur Isolierung der Klone und spektralen Analyse wurde die Biomasse der Fluoreszenz positiven Klone von den Kulturplatten entfernt und in PBS (phosphate buffed saline) resuspendiert. Der Zellaufschluss erfolgte durch Ultraschall. Nach der Klärung des Lysates durch Zentrifugation wurde die Fluoreszenz des Überstandes im Fluorometer bestimmt.

Es wurde ein fluoreszierendes Protein identifiziert. Das fluoreszierende Protein wurde als CGFP (fluorescence protein of clytia gregaria) bezeichnet. Im Folgenden wird das fluoreszierende Protein CGFP im einzelnen dargestellt.

### CGFP

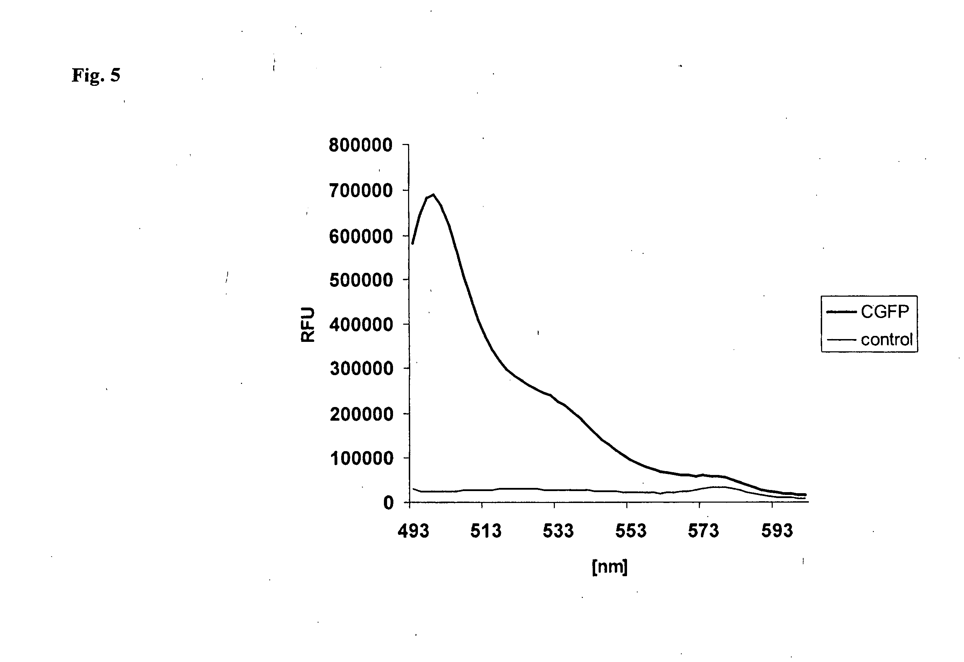

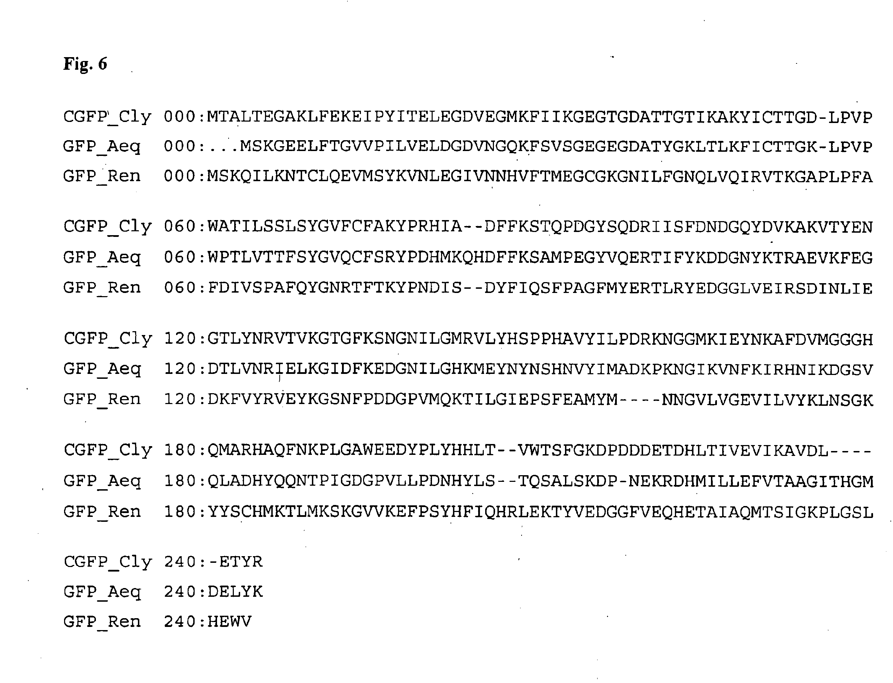

Das fluoreszierende Protein CGFP zeigt die höchste Homologie auf Aminosäureebene zu GFP aus Aequoria mit einer Identität von 44 % (gezeigt in Beispiel 8; Figur 5). Auf Nukleinsäureebene liegt die Identität unter 30 % (gezeigt in Beispiel 9; Figur 6). Zum Sequenzvergleich wurde das BLAST-Verfahren verwendet (Altschul et al., 1997).

Die Erfindung betrifft auch funktionelle Äquivalente von CGFP. Funktionelle Äquivalente sind solche Proteine, die vergleichbare physikochemische Eigenschaften haben und mindestens 70 % homolog sind. Bevorzugt ist eine Homologie von 80 % oder 90 %. Besonders bevorzugt ist eine Homologie von 95 %.

Das fluoreszierende Protein CGFP eignet sich als Reportergen für zelluläre Systeme speziell für Rezeptoren, für Ionenkanäle, für Transporter, für Transkriptionsfaktoren oder für induzierbare Systeme.

Das fluoreszierende Protein CGFP eignet sich als Reportergen in bakteriellen und eukaryotischen Systemen speziell in Säugerzellen, in Bakterien, in Hefen, in Bakulo, in Pflanzen

Das fluoreszierende Protein CGFP eignet sich als Reportergene für zelluläre Systeme in Kombination mit biolumineszenten oder chemolumineszenten Systemen speziell Systemen mit Luziferasen, mit Oxygenasen, mit Phosphatasen.

Das fluoreszierende Protein CGFP eignet sich als Markerprotein, speziell bei der FACS (Fluorescence activated cell sorter) Sortierung.

Das fluoreszierende Protein CGFP eignet sich als Fusionsprotein speziell für Rezeptoren, für Ionenkanäle, für Transporter, für Transkriptionsfaktoren, für Proteiasen, für Kinasen, für Phosphodiesterasen, für Hydrolasen, für Peptidasen, für Transferasen, für Membranproteine, für Glykoproteine.

Das fluoreszierende Protein CGFP eignet sich zur Immobilisierung speziell durch Antikörper, durch Biotin, durch magnetische oder magnetisierbare Träger.

Das fluoreszierende Protein CGFP eignet sich als Protein für Systeme des Energietransfers speziell der FRET- (Fluorescence Resonance Energy Transfer) ,BRET- (Bioluminescence Resonance Energy Transfer), FET (field effect transistors), FP (fluorescence polarization), HTRF (Homogeneous time-resolved fluorescence) Systemen.

Das fluoreszierende Protein CGFP eignet sich als Markierung von Substraten oder Liganden speziell für Proteasen, für Kinasen, für Transferasen.

Das fluoreszierende Protein CGFP eignet sich zur Expression in bakteriellen Sytemen speziell zur Titerbestimmung, als Substrate für biochemische Systeme speziell für Proteinasen und Kinasen.

Das fluoreszierende Protein CGFP eignet sich als Marker speziell gekoppelt an Antikörper, gekoppelt an Enzyme, gekoppelt an Rezeptoren, gekoppelt-an - Ionenkanäle und andere Proteine.

Das fluoreszierende Protein CGFP eignet sich als Reportergen bei der pharmakologischen Wirkstoffsuche speziell im HTS (High Throughput Screening).

Das fluoreszierende Protein CGFP eignet sich als Komponente von Detektionssystemen speziell für ELISA (enzyme-linked immunosorbent assay), für Immunohistochemie, für Western-Blot, für die konfokale Mirkoskopie.

Das fluoreszierende Protein CGFP eignet sich als Marker für die Analyse von Wechselwirkungen speziell für Protein-Protein-Wechselwirkungen, für DNA-Protein-Wechselwirkungen, für DNA-RNA-Wechselwirkungen, für RNA-RNA-Wechselwirkungen, für RNA-Protein-Wechslewirkungen (DNA : deoxyribonucleic acid; RNA : ribonucleic acid;).

Das fluoreszierende Protein CGFP eignet sich als Marker oder Fusionsprotein für die Expression in transgenen Organismen speziell in Mäusen, in Ratten, in Hamstern und anderen Säugetieren, in Primaten, in Fischen, in Würmern, in Pflanzen.

Das fluoreszierende Protein CGFP eignet sich als Marker oder Fusionsprotein zur Analyse der Embryonalentwicklung.

Das fluoreszierende Protein CGFP eignet sich als Marker über einen Kopplungsvermittler speziell über Biotin, über NHS (N-hydroxysulfosuccimide), über CN-Br.

Das fluoreszierende Protein CGFP eignet sich als Reporter gekoppelt an Nukleinsäuren speziell an DNA, an RNA.

Das fluoreszierende Protein CGFP eignet sich als Reporter gekoppelt an Proteine oder Peptide.

Das an Nukleinsäuren oder Peptiden gekoppelte fluoreszierende Protein CGFP eignet sich als Sonde speziell für Northern-Blots, für Southern-Blots, für Western-Blots, für ELISA, für Nukleinsäuresequenzierungen, für Proteinanalysen, Chip-Analysen.

Das fluoreszierende Protein CGFP eignet sich Markierung von pharmakologischen Formulierungen speziell von infektiösen Agentien, von Antikörpern, von "small molecules".

Das fluoreszierende Protein CGFP eignet sich für geologische Untersuchungen speziell für Meeres-, Grundwasser- und Flussströmungen.

Das fluoreszierende Protein CGFP eignet sich zur Expression in Expressionssystemen speziell in in-vitro Translationssystemen, in bakteriellen _Systemen, in Hefen Systemen, in Bakulo Systemen, in viralen Systemen, in eukaryotischen Systemen.

Das fluoreszierende Protein CGFP eignet sich zur Visualisierung von Geweben oder Zellen bei chirurgischen Eingriffen speziell bei invasiven, bei nicht-invasiven, bei minimal-invasiven.

Das fluoreszierende Protein CGFP eignet sich auch zur Markierung von Tumorgeweben und anderen phänotypisch veränderten Geweben speziell bei der histologischen Untersuchung, bei operativen Eingriffen.

Die Erfindung betrifft auch die Reinigung des fluoreszierenden Proteins CGFP speziell als wildtyp Protein, als Fusionsprotein, als mutagenisiertes Protein.

Die Erfindung betrifft auch die Verwendung des fluoreszierenden Proteins CGFP auf dem Gebiet der Kosmetik speziell von Badezusätzen, von Lotionen, von Seifen, von Körperfarben, von Zahncreme, von Körperpudern.

Die Erfindung betrifft auch die Verwendung des fluoreszierenden Proteins CGFP zur Färbung speziell von Nahrungsmitteln, von Badezusätzen, von Tinte, von Textilien, von Kunststoffen.

Die Erfindung betrifft auch die Verwendung des fluoreszierenden Proteins CGFP zur Färbung von Papier speziell von Grußkarten, von Papierprodukten, von Tapeten, von Bastelartikeln.

Die Erfindung betrifft auch die Verwendung des fluoreszierenden Proteins CGFP zur Färbung von Flüssigkeiten speziell für Wasserpistolen, für Springbrunnen, für Getränke, für Eis.

Die Erfindung betrifft auch die Verwendung des fluoreszierenden Proteins CGFP zur Herstellung von Spielwaren speziell von Fingerfarbe, von Schminke.

### Expression der erfindungsgemässen fluoreszierenden Proteine

Als Expression bezeichnet man die Produktion eines Moleküls, das nach dem Einbringen des Gens in eine geeignete Wirtszelle die Transcription und Translation des in einen Expressionsvektor klonierte Fremdgen erlaubt. Expressionsvektoren enthalten die für die Expression von Genen in Zellen von Prokaryoten oder Eukaryonten erforderlichen Kontrollsignale.

Expressionsvektoren können prinzipiell auf zwei verschiedene Weisen konstruiert werden. Bei den sogenannten Transcriptionsfusionen wird das vom einklonierten Fremdgen codierte Protein als authentisches, biologisch aktives Protein synthetisiert. Der Expressionsvektor trägt hierzu alle zur Expression benötigten 5'- und 3'-Kontrollsignale.

Bei den sogenannten Translationsfusionen wird das vom einklonierten Fremdgen codierte Protein als Hybridprotein zusammen mit einem anderen Protein exprimiert, das sich leicht nachweisen lässt. Die zur Expression benötigten 5'- und3'-Kontrollsignale inklusive es Startcodons und eventuell ein Teil der für die N-terminalen Bereiche des zu bildenden Hybridproteins codierenden Sequenzen stammen vom Vektor. Der zusätzliche eingeführte Proteinteil stabilisiert nicht nur in vielen Fällen das vom einklonierten Fremdgen codierte Protein vor dem Abbau durch zelluläre Proteasen, sondern lässt sich auch zum Nachweis und zur Isolierung des gebildeten Hybridproteins einsetzen. Die Expression kann sowohl transient, als auch stabil erfolgen. Als Wirtsorganismen eignen sich sowohl Bakterien, Hefen, Viren als auch eukaryotische Systeme.

### Reinigung der erfindungsgemäßen fluoreszierenden Proteine

Die Isolierung von Proteinen (auch nach Überexpression) wird häufig als Proteinreinigung bezeichnet. Zur Proteinreinigung steht eine Vielzahl an etablierten Methoden und Verfahren zur Verfügung.

Die Fest-Flüssig-Trennung ist eine Grundoperation bei Proteinisolierungen. Sowohl bei der Abtrennung der Zellen vom Kulturmedium als auch bei der Klärung des Rohextraktes nach Zellaufschluss und Entfernung der Zelltrümmer, bei der Abtrennung von Niederschlägen nach Fällungen usw. ist der Verfahrensschritt erforderlich. Er erfolgt durch Zentrifugation und Filtration.

Durch Gewinnung intrazellulärer Proteine muss die Zellwand zerstört bzw. durchlässig gemacht werden. Je nach Maßstab und Organismus werden dazu Hochdruckhomogenisatoren oder Rührwerkskugel- bzw. Glasperlenmühlen eingesetzt. Im Labormaßstab kommen u.a. mechanische Zellintegrationen und Ultraschallbehandlung zum Einsatz.

Sowohl für extrazelluläre als auch intrazelluläre Proteine (nach Zellaufschluss) sind verschiedene Fällungsverfahren mit Salzen (insbesondere Ammoniumsulfat) oder organischen Lösungsmitteln (Alkohole, Aceton) eine schnelle und defiziente Methode zur Konzentration von Proteinen. Bei der Reinigung intrazellulärer Proteine ist die Entfernung der löslichen Nucleinsäuren erstrebenswert (Fällung z.B. mit Streptomycin- oder Protaminsulfat. Bei der Gewinnung extrazellulärer Proteine werden häufig Träger (z.B. Stärke, Kieselgur) vor Zugabe der Fällungsmittel zugesetzt, um besser handhabbare Niederschläge zu erhalten.

Für die Feinreinigung stehen zahlreiche chromatographische und Verteilungsverfahren zur Verfügung (absorptions- und Ionenaustauschchromatographie, Gelfiltration, Affinitätschromatographie, Elektrophoresen. Eine Säulenchromatographie wird auch im technischen Maßstab angewandt. Für den Labormaßstab ist vor allem die Affinitätschromatographie von Bedeutung, die Reinigungsfaktoren bis zu mehreren 100 pro Schritt ermöglicht.

Extrazelluläre Proteine fallen in relativ verdünnten Lösungen an. Sie müssen ebenso wie extrazelluläre Proteine vor ihrer weiteren Verwendung konzentriert werden. Neben den schon erwähnten Verfahren hat sich - auch im industriellen Maßstab - die Ultrafiltration bewährt.

Anorganische Salze als Begleitstoffe von Proteinen sind für spezifische Anwendungen häufig unerwünscht. Sie können u.a. durch Gelfiltration, Dialyse und Diafiltration entfernt werden.

Zahlreiche Proteine kommen als Trockenpräparate zum Einsatz. Als Trocknungsverfahren sind die Vakuum-, Gefrier- und Sprühtrocknung von Bedeutung.

### Nukleotid- und Aminosäuresequenzen

Das fluoreszierende Protein CGFP wird durch die folgende Nukleotidsequenz codiert (SEQ ID NO: 1):

Daraus ergibt sich eine Aminosäuresequenz von (SEQ ID NO: 2):

Diese Sequenzen finden sich im Sequenzlisting wieder.

### Beschreibung der Figuren

Die Fig. 1 zeigt die Plasmidkarte des Vektors pTriplEX2-CGFP .
Die Fig. 2 zeigt die Plasmidkarte des Vektors pcDNA3-CGFP .
Die Fig. 3 zeigt die transiente Emission von CGFP in CHO-Zellen im Expressionsvector pcDNA3-CGFP. Die Figur zeigt die mikroskopische Aufnahme der transient transfizierten Zellen bei einer Anregung von 480 nm und einer Emission von 520 nm.
Die Fig. 4 zeigt die Exitation des CGFP und des Kontrolllysates
Die Fig. 5 zeigt die Emission des CGFP und des Kontrolllysates
Die Fig. 6 zeigt das Aligment von CFGP, GFP (Aquoria) und GFP (Renilla) auf Aminosäureebene.

| | |
|---|---|
| CGFP_Cly : | CGFP aus Clytia gregaria |
| GFP_Ren : | GFP aus Renilla |
| GFP_Aeq . | GFP aus Aequoria |

Die Fig. 7 zeigt das Aligment von CFGP, GFP (Aquoria) und GFP (Renilla) auf Nukleinsäureebene.

| | |
|---|---|
| CGFP_ Cly: | CGFP aus Clytia gregaria |
| GFP_Ren : | GFP aus Renilla |
| GFP_Aeq . | GFP aus Aequoria |

### Beispiele

### Beispiel 1

Als Vektor zur Herstellung des im folgenden dargestellten Konstruktes wurde das Plasmid pTriplEx2 der Firma Clontech verwendet. Das Derivat des Vektors wurde als pTriplEx2-CGFP bezeichnet. Der Vektor pTriplEx2-CGFP wurde zur Expression von CGFP in bakteriellen Systemen verwendet.

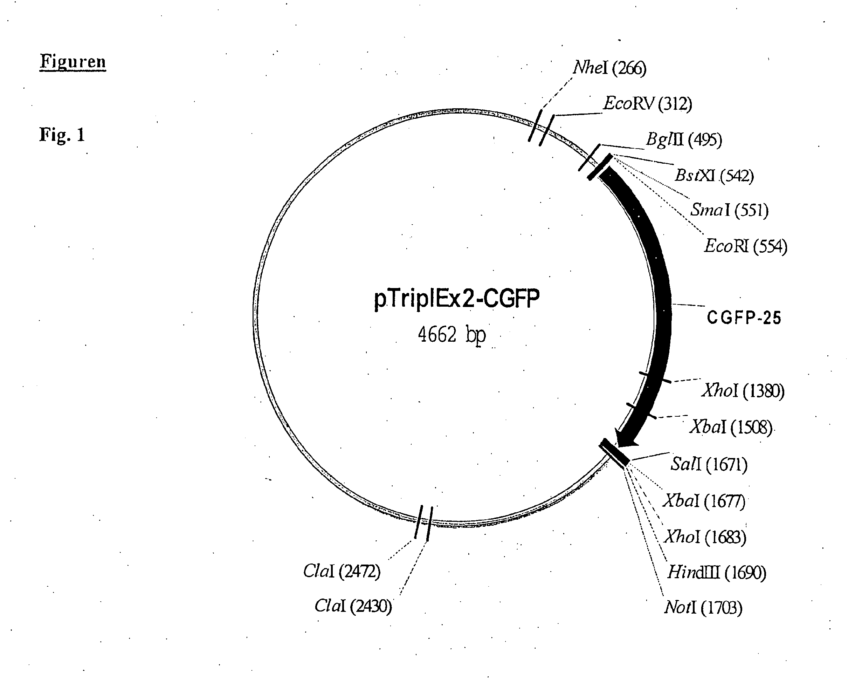

Die Fig. 1 zeigt die Plasmidkarte des Vektors pTrip1EX2-CGFP.

### Beispiel 2

Als Vektor zur Herstellung des im folgenden dargestellten Konstruktes wurde das Plasmid pcDNA3.1(+) der Firma Clontech verwendet. Das Derivat des Vektors wurde als pcDNA3-CGFP bezeichnet. Der Vektor pcDNA3-CGFP wurde zur Expression von CGFP in eukaryotischen Systemen verwendet.

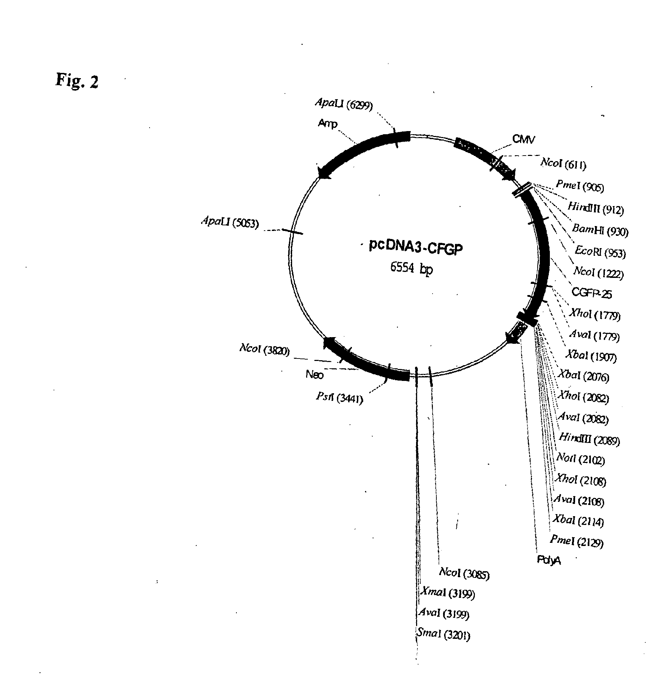

Die Fig. 2 zeigt die Plasmidkarte des Vektors pcDNA3-CGFP .

### Beispiel 3

### Bakterielle Expression

Die bakterielle Expression erfolgte im E. coli Stamm BL21(DE3) durch Transformation der Bakterien mit den Expressionsplasmiden pTriplEX2-CGFP und pTrip1EX2. Die transformierten Bakterien wurden in LB-Medium bei 37°C für 3 Stunden inkubiert und die Expression für 4 Stunden durch Zugabe von IPTG bis zu einer Endkonzentration von 1 mM induziert. Die induzierten Bakterien wurden durch Zentrifugation geerntet, in PBS resuspendiert und durch Ultraschall aufgeschlossen. Die Fluoreszenz wurde mit Hilfe eines Fluorometers bestimmt.

### Beispiel 4

### Eukaryotische Expression

Die konstitutive eukaryotische Expression erfolgte in CHO-Zellen durch Transfektion der Zellen mit den Expressionsplasmiden pcDNA3-CGFP und pcDNA3.1 (+) in transienten Experimenten. Hierzu wurden 10000 Zellen pro Loch in DMEM-F12 Medium auf 96 Loch Mikrotiterplatten plattiert und über Nacht bei 37°C inkubiert. Die Transfektion erfolgte mit Hilfe des Fugene 6 Kits (Roche) nach Herstellerangaben. Die transfizierten Zellen wurden über Nacht bei 37°C in DMEM-F12 Medium inkubiert. Die Messung der Fluoreszenz erfolgte im Fluorometer bei Raumtemperatur.

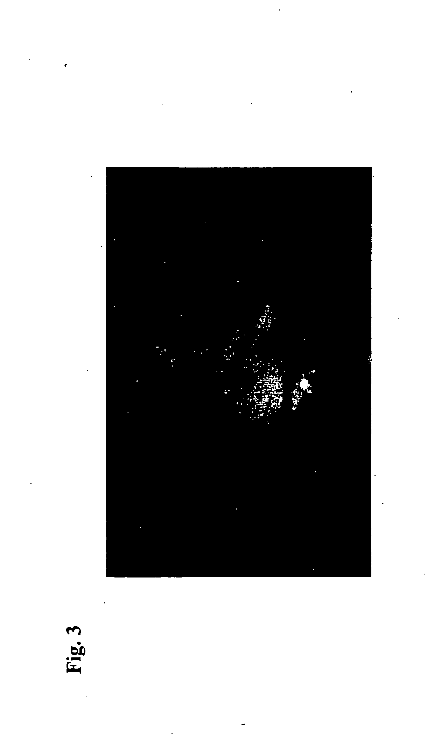

Die Figur 3 zeigt die Expression von CGFP in CHO-Zellen.

### Beispiel 5

### Spektrum des fluoreszierenden Proteins CGFP

Zur Messung des Emissionsspektrums wurden E. coli BL21(DE3) mit den Plasmiden pTriplEX2=CGFP und pTrip1EX2 transformiert. Die Induktion erfolgte durch die Zugabe von 1 mM IPTG und einer Inkubation von 4 Stunden bei 37°C. Anschließend wurden die Bakterien geerntet und in PBS resuspendiert. Die Lyse erfolgte durch Ultraschall. Anschließend erfolgte die Messung der Fluoreszenz im Fluorometer.

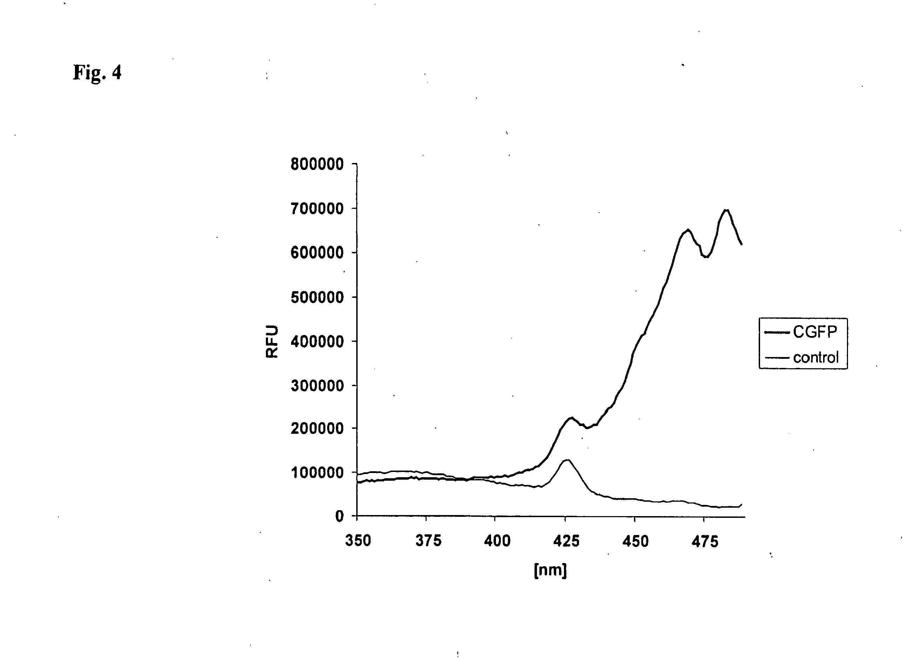

Die Figur 4 zeigt die Exitation des CGFP und des Kontrolllysates

Die Figur 5 zeigt die Emission des CGFP und des Kontrolllysates

### Beispiel 6

### BLAST

Ergebnis einer BLAST-Analyse von CFGP auf der Aminosäureebene.
>AA2002:ABB06186 Abb06186 Green fluorescent protein GFPxml9 SEQ ID, NO:15. 5/2002, Length = 271, Score = 219 bits (558), Expect = 3e-56, Identities = 102/228 (44%), Positives = 151/228 (65%), Gaps = 3/228 (1%)
>gb|AAK02065.1| mutant green fluorescent protein [synthetic construct], Length = 238, Score = 219 bits (557), Expect = 4e-56, Identities = 102/227 (44%), Positives = 150/227 (65%), Gaps = 3/227 (1%)
>gb|AAL33915.1|AF435430_1 green fluorescent protein [Aequorea macrodactyla], Length = 238, Score = 218 bits (556), Expect = 5e-56, Identities = 102/227 (44%), Positives = 150/227 (65%), Gaps = 3/227 (1%)
>gb|AAL33918.1|AF435433_1 green fluorescent protein [Aequorea macrodactyla], Length = 238, Score = 218 bits (555), Expect = 7e-56, Identities = 101/227 (44%), Positives = 149/227 (65%), Gaps = 3/227 (1%)
>gb|AAL33916.1|AF435431_1 green fluorescent protein [Aequorea macrodactyla], Length = 238, Score = 218 bits (554), Expect = 9e-56
   Identities = 102/227 (44%), Positives = 150/227 (65%), Gaps = 3/227 (1%)
>gb|AAL33917.1|AF435432_1 orange fluorescent protein [Aequorea macrodactyla], Length = 238, Score = 218 bits (554), Expect = 9e-56, Identities = 101/227 (44%), Positives = 149/227 (65%), Gaps = 3/227 (1%)
>AA2002:ABB06185 Abb06185 Green fluorescent protein GFPxm18 SEQ ID, NO:13. 5/2002, Length = 271, Score = 217 bits (552), Expect = le-55, Identities = 101/228 (44%), Positives = 151/228 (65%), Gaps = 3/228 (1%)
>AA2002:ABB06184 Abb06184 Green fluorescent protein GFPxm16 SEQ ID, NO:11. 5/2002, Length = 271, Score = 216 bits (551), Expect = 2e-55, Identities = 101/228 (44%), Positives = 150/228 (65%), Gaps = 3/228 (1%)
>AA2002:ABB06181 Abb06181 Green fluorescent protein GFPxm SEQ ID, NO:5. 5/2002, Length = 271, Score = 216 bits (551), Expect = 2e-55, Identities = 101/228 (44%), Positives = 150/228 (65%), Gaps = 3/228 (1%)
>gb|AAL33912.1|AF435427_1 green fluorescent protein [Aequorea macrodactyla], Length = 238, Score = 216 bits (551), Expect = 2e-55, Identities = 101/227 (44%), Positives = 150/227 (65%), Gaps = 3/227 (1%)
>gb|AAK02064.1| mutant green fluorescent protein [synthetic construct], Length = 238, Score = 216 bits (551), Expect = 2e-55, Identities = 101/227 (44%), Positives = 150/227 (65%), Gaps = 3/227 (1%)

### Beispiel 7

### BLAST

Ergebnis einer BLAST-Analyse von CFGP auf Nukleinsäureebene.
>gb|AF468563.1| Crassostrea gigas clone c077 microsatellite sequence, Length = 415, Score = 41.1 bits (21), Expect = 1.4, Identities = 25/27 (92%)
>gb|AC079685.2| Oryza sativa chromosome 10 clone OSJNBb0012A20, complete sequence, Length = 131599, Score = 41.1 bits (21), Expect = 1.4, Identities = 27/30 (90%)
>gb|AF427906.1|AF427906 Solenopsis globularia littoralis putative odorant binding protein, precursor (Gp-9) gene, complete cds, Length = 1767, Score = 41.1 bits (21), Expect = 1.4, Identities = 23/24 (95%)
>gb|AF297617.1|AF297617 Echinococcus granulosus genotype 1 mitochondrion, complete genome, Length = 13588, Score = 41.1 bits (21), Expect = 1.4, Identities = 23/24 (95%)

### Beispiel 8

Die Figur 6 zeigt das Aligment von CGFP, GFP (Aquoria) und GFP (Renilla) auf Aminosäureebene.

### Beispiel 9

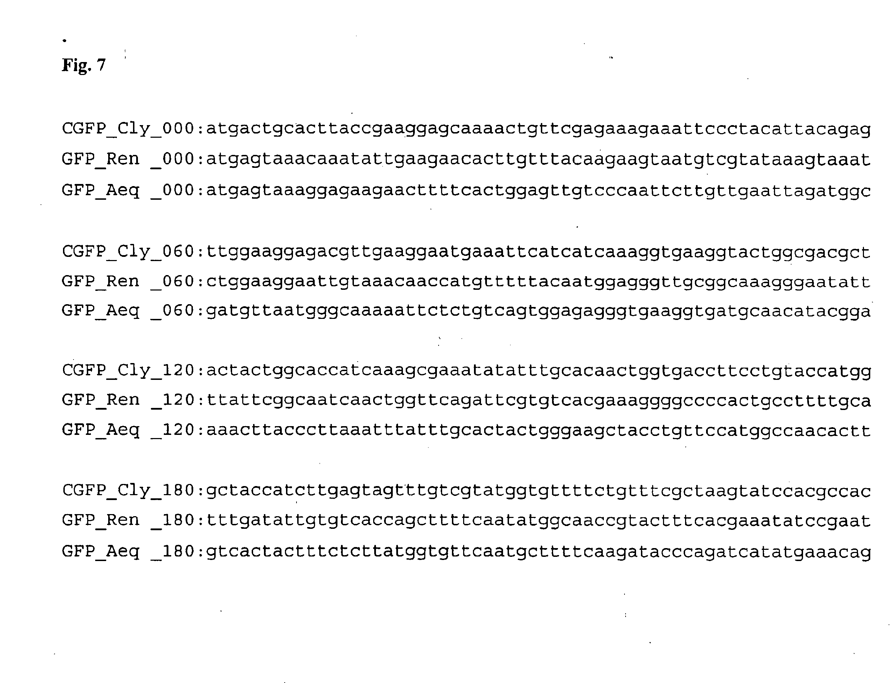

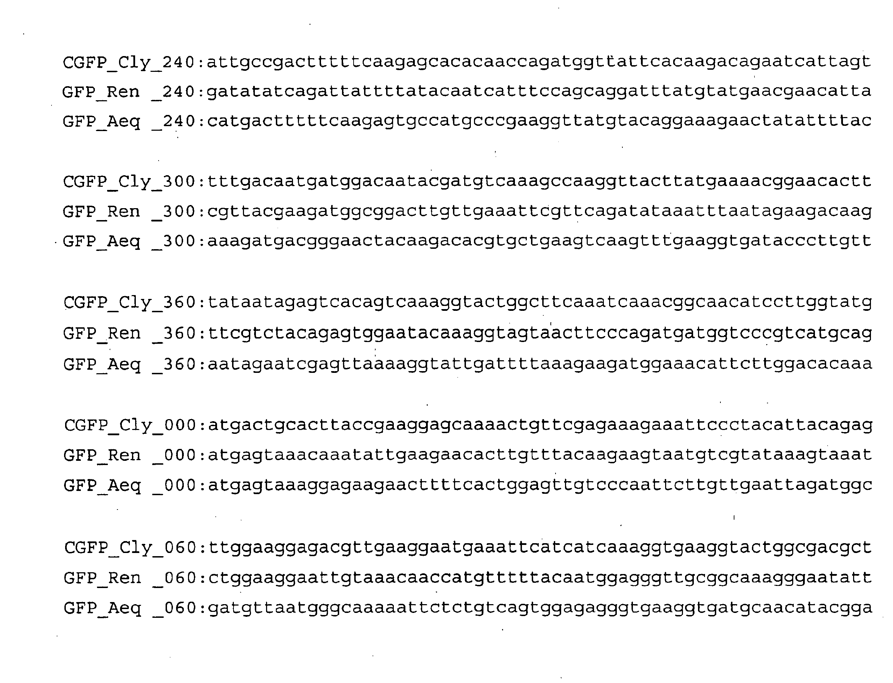

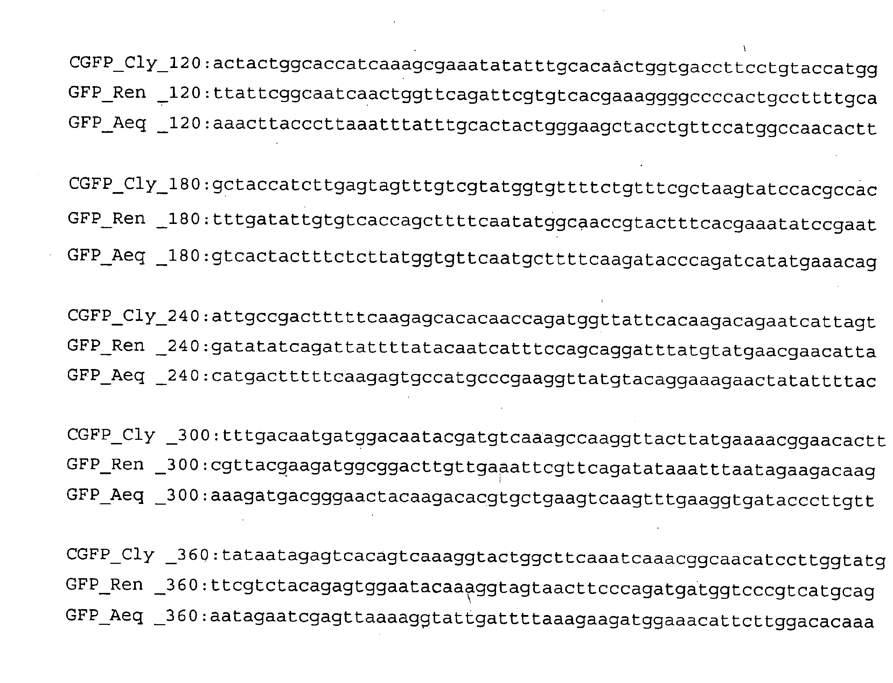

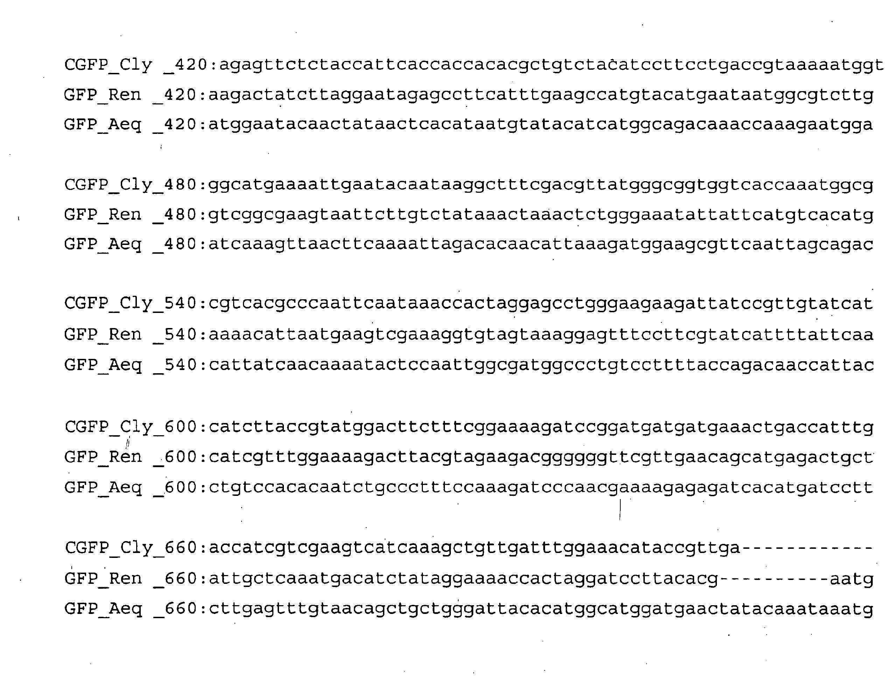

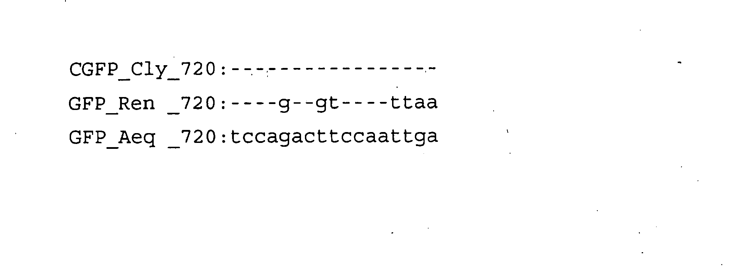

Die Figur 7 zeigt das Aligment von CGFP, GFP (Aquoria) und GFP (Renilla) auf Nukleinsäureebene.

### Literatur / Patente

US patent no. 4,777,128
US patent no. 4,927,923
US patent no. 5,162,508
US patent no. 5,279,943
US patent no. 5,958,713
US patent no. 6,172,188
US patent no. 6,232,107
US patent no. 6,436,682
WO19962389
WO199711094
WO 199728261
WO1998/02571
WO199949019
WO200071565
WO200134824
WO200168824
WO200257451
Alam J, Cook JL. Reporter genes: application to the study of mammalian gene transcription. Anal Biochem. 1990 Aug 1;188(2):245-54
Altschul, Stephen F., Thomas L. Madden, Alejandro A. Schäffer, Jinghui Zhang, Zheng Zhang, Webb Miller, and David J. Lipman (1997); Gapped BLAST and PSI-BLAST: a new generation of protein database search programs; Nucleic Acids Res. 25:3389-3402
Cardullo et al. (1988) Detection of nucleic acid hybridization by nonradiative fluorescence resonance energy transfer.; Proc. Natl. Acad. Sci. U.S.A. 85 : 8790-8794
Cormier, M.J., Hori, K., Karkhanis, Y.D., Anderson, J.M., Wampler, J.E., Morin, J.G., and Hastings, J.W. (1973) Evidence for similar biochemical requirements for bioluminescence among the coelenterates. J. Cell. Physiol. 81, 291-298.
Cormier, M.J., Hori, K., and Anderson, J.M. (1974) Bioluminescence in coelenterates. Biochim. Biophys. Acta 346, 137-164.
Cullen Bryan R., Malim Michael H., Secreted placental alkaline phosphatase as a eukaryotic reporter gene. Methods in Enzymology, 216:362ff
Davenport, D. and Nicol, J.A.C. (1955) Luminescence in Hydromedusae. Proc. R. Soc. B 144, 399-411.
Delagrave et al., Red-shifted excitation mutants of the green fluorescent protein, Bio/Technology 13(2):151-154 (1995)
Ehrig et al., Green-fluorescent protein mutants with altered fluorence excitationspectra, FEBS Letters 367:163-166 (1995)
Hastings, J.W. and Morin, J.G. (1969) Comparative biochemistry of calciumactivated photoproteins from the ctenophore, Mnemiopsis and the coelenterates Aequorea, Obelia, and Pelagia. Biol. Bull. 137, 402.
Heim et al., (1996) Engineering green fluorescent protein for improved brightness, longer wavelengths and fluorescence resonance energy transfer, Current Biology 6(2):178-182 (1996).
Inouye S, Tsuji FI. (1994) Aequorea green fluorescent protein. Expression of the gene and fluorescence characteristics of the recombinant protein. FEBS Lett 1994 Mar 21;341(2-3):277-80
Johnson, F.H., Shimomura, O., Saiga, Y., Gershman, L.C., Reynolds, G.T., and Waters, J.R. (1962) Quantum efficiency of Cypridina luminescence, with a note on that of Aequorea. J. Cell. Comp. Physiol. 60, 85-103.
Krieg, S., Castles, C., Allred, D., Benedix, M., Fuqua S., RNA from air-dried frozen sections for RT-PCR and differential display. Biotechniques, 1996 Sep;21(3):425-8.
Levine, L.D. and Ward, W.W. (1982) Isolation and characterization of a photoprotein, "phialidin", and a spectrally unique green-fluorescent protein from the bioluminescent jellyfish Phialidium gregarium. Comp. Biochem. Physiol. 72B, 77-85.
Mitra et al., Fluorescence resonance energy tranfer between blue-emitting and red-shifted excitation derivatives of the green fluorescent protein, Gene 73(1):13-17 (1996).
Morin, J.G. and Hastings, J.W. (1971) Biochemistry of the bioluminescence of colonial hydroids and other coelenterates. J. Cell. Physiol. 77, 305-311.
Morin, J.G. and Hastings, J.W. (1971) Energy transfer in bioluminescent system. J Cell. Physiol. 77, 313-318.
Phillips GN. Structure and dynamics of green fluorescent protein. Curr Opin Struct Biol. 1997 Dec;7(6):821-7
Shimomura O., Bioluminescence in the sea: photoprotein systems. Symp Soc Exp Biol. 1985;39:351-72.
Snowdowne KW, Borle AB. Measurement of cytosolic free calcium in mammalian cells with aequorin. Am J Physiol. 1984 Nov;247(5 Pt 1):C396-408.
Ward, W.W. (1998) Biochemical and physical properties of green fluorescent protein. In: Green Fluorescent Protein: Properties, Applications, and Protocols (Chalfie, M. and Kain, S., eds) pp. 45-70. Wiley-Liss, Inc.
Ward et al., Energy Transfer Via Protein-Protein Interation in Renilla Bioluminescence Photochemistry and Photobiology 27:389-396 (1978).
Wampler, J.E., Hori, K., Lee, J.W., and Cormier, M.J. (1971) Structured bioluminescence. Two emitters during both the in vitro and the in vivo bioluminescence of the sea pansy, Renilla, Biochemistry 10, 2903-2909.
Wampler, J.E., Karkhanis, Y.D., Morin, J.G., Cormier, M.J. (1973) Similarities in the bioluminescence from the Pennatulacea. Biochim. Biophys. Acta 314, 104-109.
Yang Te-Tuan, Sinai Parisa, Kitts Paul A. Kain Seven R., Quantification of gene expresssion with a secreted alkaline phosphatase reporter system. Biotechnique, 1997 23(6) 1110ff

## Patentansprüche

1. Nukleinsäuremolekül, ausgewählt aus der Gruppe bestehend aus
a) Nukleinsäuremolekülen, die das Polypeptid offenbart durch SEQ ID NO: 2 kodieren;
b) Nukleinsäuremolekülen, welche die durch SEQ ID NO: 1 dargestellte Sequenz enthalten;
c) Nukleinsäuremolekülen, deren komplementärer Strang mit einem Nukleinsäuremolekül aus a) oder b) unter stringenten Bedingungen hybridisiert und welche fluoreszierende Proteine kodieren;
d) Nukleinsäuremolekülen, welche sich auf Grund der Degenerierung des genetischen Kodes von den unter c) genannten unterscheiden;
e) Nukleinsäuremolekülen, welche eine Sequenzhomologie von mindestens 95% zu SEQ ID NO: 1 zeigen, und welche fluoreszierende Proteine kodieren; und
f) Nukleinsäuremolekülen, welche eine Sequenzhomologie von mindestens 65% zu SEQ ID NO: 1 zeigen, und welche fluoreszierende Proteine kodieren.

2. Moleküle nach Anspruch 1, bei denen die Sequenz einen funktionalen Promotor 5' zur Sequenz enthält.

3. Moleküle nach Anspruch 2, die Bestandteil von rekombinanten DNA oder RNA Vektoren sind.

4. Organismen, die einen nach Anspruch 3 beschriebenen Vektor enthalten.

5. Oligonukleotide mit mehr als 10 aufeinanderfolgenden Nukleotiden, die identisch oder komplementär zu DNA oder RNA Sequenzen nach Anspruch 1 sind.

6. Peptide, die durch die Nukleotidesequenzen nach Anspruch 1 kodiert sind.

7. Verfahren zur Expression der Clytia Green Fluorescent Protein (CGFP) Polypeptide gemäss Anspruch 6 in Bakterien, eukaryontischen Zellen oder in *in vitro* Expressionssystemen.

8. Verfahren zur Aufreinigung/Isolierung eines CGFP Polypeptides gemäss Anspruch 6.

9. Peptide mit mehr als 5 aufeinanderfolgenden Aminosäuren, die immunologisch durch Antikörper gegen das fluoreszierende Protein CGFP erkannt werden.

10. Verwendung des fluoreszierenden Proteins CGFP gemäß den Ansprüchen 1 bis 7 als Marker- und Reportergen.

## Claims

1. Nucleic acid molecule, selected from the group consisting of
a) nucleic acid molecules encoding the polypeptide disclosed by SEQ ID NO: 2;
b) nucleic acid molecules containing the sequence depicted by SEQ ID NO: 1;
c) nucleic acid molecules whose complementary strand hybridizes under stringent conditions with a nucleic acid molecule of a) or b) and which encode fluorescent proteins;
d) nucleic acid molecules which differ from those mentioned under c) due to the degeneracy of the genetic code;
e) nucleic acid molecules whose sequences are at least 95% homologous to SEQ ID NO: 1 and which encode fluorescent proteins; and
f) nucleic acid molecules whose sequences are at least 65% homologous to SEQ ID NO: 1 and which encode fluorescent proteins.

2. Molecules according to Claim 1, whose sequence contains a functional promoter 5' of the sequence.

3. Molecules as claimed in Claim 2, which are part of recombinant DNA or of RNA vectors.

4. Organisms, which contain a vector described as claimed in Claim 3.

5. Oligonucleotides, having more than 10 contiguous nucleotides which are identical or complementary to DNA or RNA sequences as claimed in Claim 1.

6. Peptides, which are encoded by the nucleotide sequences as claimed in Claim 1.

7. Method of expressing the *Clytia* green fluorescent protein (CGFP) polypeptides as claimed in Claim 6 in bacteria, eukaryotic cells or in *in vitro* expression systems.

8. Method of purifying/isolating a CGFP polypeptide as claimed in Claim 6.

9. Peptides, having more than 5 contiguous amino acids which are recognized immunologically by antibodies to the fluorescent protein CGFP.

10. Use of the fluorescent protein CGFP as claimed in Claims 1 to 7 as a marker gene and reporter gene.

## Revendications

1. Molécule d'acide nucléique, sélectionnée dans le groupe constitué de
a) molécules d'acide nucléique, qui codent le polypeptide divulgué par SEQ ID NO:2;
b) molécules d'acide nucléique, qui contiennent la séquence représentée par SEQ ID NO:1;
c) molécules d'acide nucléique dont le brin complémentaire hybride avec une molécule d'acide nucléique de a) ou b) dans des conditions contraignantes et qui codent des protéines fluorescentes;
d) molécules d'acide nucléique, qui se distinguent de celles qui sont mentionnés sous c) en raison de la dégénérescence du code génétique;
e) molécules d'acide nucléique, qui présentent une homologie de séquence d'au moins 95 % par rapport à SEQ ID NO:1 et qui codent des protéines fluorescentes et
f) molécules d'acide nucléique, qui présentent une homologie de séquence d'au moins 65 % par rapport à SEQ ID NO:1 et qui codent des protéines fluorescentes.

2. Molécules selon la revendication 1 dans lesquelles la séquence contient un promoteur fonctionnel 5' de la séquence.

3. Molécules selon la revendication 2 qui font partie de l'ADN ou qui sont des vecteurs de l'ARN.

4. Organismes qui contiennent un vecteur selon la revendication 3.

5. Oligonucléotides comportant plus de 10 nucléotides consécutifs, qui sont identiques aux ou complémentaires des séquences d'ADN ou d'ARN selon la revendication 1.

6. Peptides qui sont codés par les séquences de nucléotides selon la revendication 1.

7. Procédé d'expression des polypeptides de Clytia Green Fluorescent Protein (CGFP) selon la revendication 6 dans des bactéries, des cellules eucaryotes ou des systèmes d'expression in vitro.

8. Procédé de purification/d'isolation d'un polypeptide CGFP selon la revendication 6.

9. Peptides comportant plus de 5 acides aminés consécutifs, qui sont immunologiquement reconnus par des anticorps dirigés contre la protéine fluorescente CGFP.

10. Utilisation de la protéine fluorescente CGFP conformément aux revendications 1 à 7 comme gène marqueur et rapporteur.
